(19) Europäisches Patentamt
European Patent Office
Office européen des brevets

(11) Numéro de publication : **0 063 075**
**B1**

(12) **FASCICULE DE BREVET EUROPÉEN**

(45) Date de publication du fascicule du brevet : **04.07.84**

(51) Int. Cl.³ : **C 07 D295/10**, A 61 K 31/445

(21) Numéro de dépôt : **82400577.1**

(22) Date de dépôt : **30.03.82**

(54) **(2,6-Diméthoxy-4-hydroxyphényl)-(3-pipéridinopropyl)-cétone et ses sels d'addition, utilisation en thérapeutique et procédé de préparation.**

(30) Priorité : **15.04.81 FR 8107597**

(43) Date de publication de la demande : **20.10.82 Bulletin 82/42**

(45) Mention de la délivrance du brevet : **04.07.84 Bulletin 84/27**

(84) Etats contractants désignés : **AT BE CH DE FR GB IT LI LU NL SE**

(56) Documents cités :
**FR-A- 2 092 133**
**FR-A- 2 134 218**
**FR-A- 2 404 003**

(73) Titulaire : **LABORATOIRE L. LAFON Société anonyme dite: 1 rue Georges Médéric F-94701 Maisons-Alfort (FR)**

(72) Inventeur : **Lafon, Louis 5 rue de l'Alboni F-75016 Paris (FR)**

(74) Mandataire : **Combe, André et al CABINET BEAU DE LOMENIE 55 rue d'Amsterdam F-75008 Paris (FR)**

**EP 0 063 075 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne, en tant que produits industriels nouveaux appartenant à la famille des (alkoxy- et hydroxy-phényl)-(3-aminopropyl)-cétones, la (2,6-diméthoxy-4-hydroxy-phényl)-(3-pipéridinopropyl)-cétone et ses sels d'addition. Elle concerne également leur utilisation en thérapeutique et leur procédé de préparation.

On sait que dans le passé on a déjà décrit et proposé en thérapeutique un certain nombre de composés du type (alkoxy- et hydroxy-phényl)-(aminoalkyl)-cétone. En particulier, on connaît :

— du brevet français n° 1.492.256, du brevet français (BSM) n° 5636M et de l'article de A. BOUCHERLE et al., Chimie Thérapeutique, *3* (n° 4), 256-259 (1968), les (2,4,6-triméthoxyphényl)-(2-pipéridinoéthyl)-cétone et (2,4,6-triméthoxyphényl)-[2-(4-méthylpipéridino)-éthyl]-cétone, qui présentent des effets essentiellement antiinflammatoires, antalgiques et antipyrétiques ;

— du brevet britannique n° 1.115.992, les (2,4-diméthoxyphényl)-(pipéridinométhyl)-cétone et (2,4,6-triméthoxyphényl-[(4-méthylpipéridino)-méthyl]-cétone, qui présentent des effets essentiellement antispasmodiques et tranquillisants ; et

— du brevet britannique n° 1.325.192, le chlorhydrate de (2,4,6-trihydroxyphényl)-(3-pipéridinopropyl)-cétone (n° de code LL 1647), qui présente des effets essentiellement antispasmodiques et qui est utile dans le traitement des coliques néphrétiques, et le chlorhydrate de (2,4,6-triméthoxyphényl)-(3-pyrrolidinopropyl)-cétone (n° de code LL 1656), qui est un agent vasodilatateur périphérique de référence, qui a fait l'objet d'une publication par DEBRAY et al., Thérapie, *30,* 259-266 (1975) et qui est commercialisé en pharmacie sous la désignation de FONZYLANE (Dénomination Commune Internationale : CHLORHYDRATE de BUFLOMEDIL).

On sait enfin du brevet français n° 2 404 003 que le remplacement d'un ou plusieurs groupes $OCH_3$ du LL 1656 précité par un ou plusieurs groupes OH a des conséquences imprévisibles en ce qui concerne les éventuelles propriétés vasodilatatrices périphériques des produits résultants. En effet, le brevet français n° 2 404 003 montre qu'il n'y a pas de relation structure-activité dans ledit remplacement $OCH_3$/OH : si les (2,4,6-trihydroxyphényl-(3-pyrrolidinopropyl)-cétone et (2,4-diméthoxy-6-hydroxyphényl)-(3-pyrrolidinopropyl)-cétone sont des agents vasodilatateurs périphériques aussi intéressants que le LL 1656, en revanche le dérivé (2,6-dihydroxy-4-méthoxyphényl)- ou (2,4-dihydroxy-6-méthoxyphényl)-(3-pyrrolidinopropyl)-cétone et le dérivé (2,6-diméthoxy-4-hydroxyphényl)-(3-pyrrolidinopropyl)-cétone sont dépourvus d'effets vasodilatateurs périphériques.

On vient de trouver de façon surprenante que la (2,6-diméthoxy-4-hydroxyphényl)-(3-pipéridinopropyl)-cétone et ses sels d'addition sont, en tant qu'agents vasodilatateurs périphériques, (i) plus efficaces que les produits analogues du type pipéridino et (ii) au moins aussi intéressants que le LL 1656 précité.

Un nouveau dérivé appartenant à la famille des alkoxy- et hydroxy-phényl)-(3-aminopropyl)-cétones selon l'invention est caractérisé en ce qu'il est choisi parmi l'ensemble constitué par

(i) la (2,6-diméthoxy-4-hydroxyphényl)-(3-pipéridinopropyl)-cétone de formule :

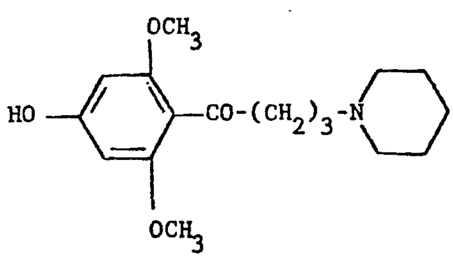

(I)

et

(ii) ses sels d'addition.

Par sels d'addition, on entend ici les sels d'addition d'acides (obtenus par réaction de la base libre de formule (I) avec un acide minéral ou organique) et les sels d'ammonium. Parmi les acides utilisables pour salifier la base de formule (I), on peut notamment citer les acides chlorhydrique, bromhydrique, nitrique, sulfurique, acétique, propionique, oxalique, fumarique, maléique, succinique, benzoïque, cinnamique, mandélique, citrique, lactique, malique, tartrique, aspartique, p-toluènesulfonique et méthanesulfonique. Parmi les composés permettant d'obtenir des sels d'ammonium, on peut citer notamment $ICH_3$ et $ClCH_3$. Les sels d'addition d'acides sont d'une manière générale préférés aux sels d'ammonium.

Le produit préféré selon l'invention est le chlorhydrate de (2,6-diméthoxy-4-hydroxyphényl)-(3-pipéridinopropyl)-cétone.

La base libre de formule (I) peut être préparée selon une méthode connue en soi par application de mécanismes réactionnels classiques. Le procédé préconisé selon l'invention consiste à faire réagir stoechiométriquement le 1,5-diméthoxy-3-hydroxy-benzène de formule :

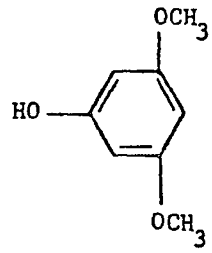

(II)

avec le pipéridinobutyronitrile de formule :

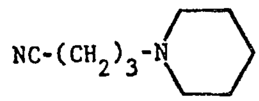

(III)

en présence de $AlCl_3$ et en faisant passer au sein du milieu réactionnel pendant au moins 2 h à une température de 10-20 °C un courant gazeux de HCl, puis à hydrolyser le dérivé cétimine ainsi formé et qui a pour formule :

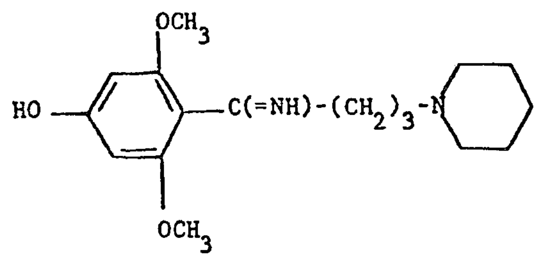

(IV)

pour obtenir le composé de formule (I).

Dans ce procédé, la réaction de II avec III est effectuée avantageusement dans le chlorobenzène en mettant en œuvre 1 mole de II, 1 mole de III et 1 mole de $AlCl_3$. Il n'est pas nécessaire d'isoler le dérivé cétimine IV : l'hydrolyse de celui-ci est effectuée directement dans le milieu réactionnel résultant de la réaction de II avec III en introduisant dans ledit milieu réactionnel de la glace. On recueille la phase aqueuse résultante par décantation, puis on la traite à l'ébullition pendant au moins 30 min en présence de noir de carbone.

Selon l'invention, on préconise également une composition thérapeutique qui est notamment utile dans le traitement des maladies vasculaires, en particulier le traitement des escarres, et qui est caractérisée en ce qu'elle renferme, en association avec un excipient physiologiquement acceptable, la base libre de formule (I) ou l'un de ses sels d'addition non toxiques. Bien entendu, une telle composition thérapeutique comprendra une quantité pharmaceutiquement efficace d'ingrédient actif vasodilatateur périphérique.

D'autres avantages et caractéristiques de l'invention seront mieux compris à la lecture qui va suivre d'exemples de préparation nullement limitatifs mais donnés à titre d'illustration.

## Préparation I

Obtention du chlorhydrate de (2,6-diméthoxy-4-hydroxyphényl)-(3-pipéridinopropyl)-cétone

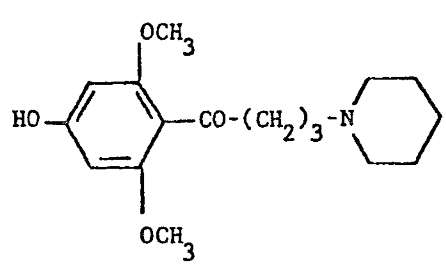

, HCl

(Exemple 1 ; n° de code CRL 40746)

On introduit dans un ballon tricol muni d'une agitation 30,8 g (0,2 mole) de 1,5-diméthoxy-3-hydroxybenzène, 34 g (0,2 mole) de pipéridinobutyronitrile et 200 ml de chlorobenzène ; on ajoute alors lentement 28 g (0,2 mole) de chlorure d'aluminium puis, en maintenant la température entre 14 et 20 °C, on fait passer au sein du milieu réactionnel résultant sous agitation un courant gazeux de HCl anhydre

pendant 4,25 h. On laisse reposer à 4 °C pendant 20 h puis jette sur de la glace (pour hydrolyser le chlorhydrate de cétimine qui a été formé) et décante la phase aqueuse inférieure que l'on fait bouillir 1 h en présence de noir CXA. On filtre à chaud et refroidit la liqueur mère. On obtient un produit cristallisé et une huile. (L'huile après isolement et traitement donnera le CRL 40747 (voir préparation II ci-dessous). Le produit cristallisé est filtré, lavé sur filtre avec de l'eau glacée pour le débarrasser de l'huile qui l'accompagne. Par recristallisation dans l'eau, on obtient un hydrate cristallisé de couleur jaune qui, après trituration dans l'éthanol, donne 21 g (rendement 31 %) de CRL 40746 sous la forme d'une poudre cristalline blanche, soluble dans l'eau. F. inst. = 214-215 °C (avec décomposition).

Préparation II

Obtention du chlorhydrate de (2,4-diméthoxy-6-hydroxyphényl)-(3-pipéridinopropyl)-cétone

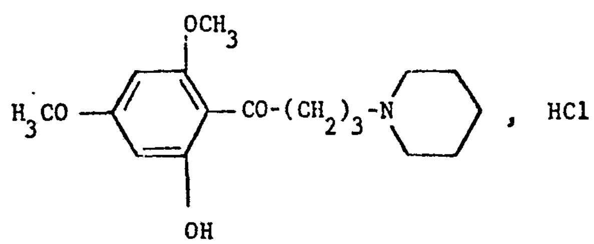

(Exemple comparatif CP1, n° de code CRL 40747)

L'huile qui décante dans la préparation I ci-dessus, après hydrolyse du chlorhydrate de cétimine IV puis traitement à l'ébullition de la phase aqueuse en présence de noir de carbone, est extraite au chloroforme. La phase chloroformique est séchée sur $Na_2SO_4$ en présence de noir de carbone. On évapore le solvant et obtient une poudre grossière de couleur jaune que l'on recristallise du mélange isopropanol-eau (100 : 2) v/v. On obtient 18,2 g (rendement 26,6 %) de CRL 40747 qui se présente sous la forme d'une poudre cristalline de couleur crème pâle très soluble dans l'eau. F. inst. = 181-183 °C (avec décomposition).

On a résumé ci-après une partie des résultats des essais qui ont été entrepris chez l'animal et plus précisément ceux des essais comparatifs relatifs aux produits consignés dans le tableau I ci-après, à savoir le CRL 40746 (exemple 1), ses analogues structuraux (CP1-CP6) et un produit vasodilatateur périphérique de référence qui est le LL 1656 (CP7) précité.

Les propriétés vasodilatatrices périphériques ont été étudiées chez le chien mâle anesthésié au nembutal (6 animaux par dose et par produit). Les produits à comparer sont administrés en solution dans du sérum physiologique sous un volume de 6 ml/animal par voie intraveineuse (perfusion de 1 ml/min) et sous un volume de 10 ml/animal par voie intraduodénale. Par rapport aux témoins (les mêmes animaux ne recevant que le sérum physiologique), on mesure trois paramètres : la pression artérielle moyenne (exprimée en mm Hg ; 1 mm Hg correspond à $1,333\,224 \times 10^2$ Pa), la fréquence cardiaque (exprimée en battements/minute) et le débit artériel fémoral (exprimé en ml/min). Les variations de ces paramètres exprimées en pourcentages, par rapport aux témoins, sont données dans les tableaux II (administration intraveineuse) et III (administration intraduodénale) ci-après.

La vasodilatation périphérique se traduit dans ces circonstances par une augmentation du débit artériel fémoral. On dit, selon les présentes conditions opératoires, qu'un produit est un agent vasodilatateur périphérique si le débit artériel fémoral augmente d'au moins 30 % par administration intraveineuse, d'une part, et par administration intraduodénale, d'autre part, sans que la variation de la pression artérielle soit supérieure à + 10 % ou inférieure à − 10 %.

Les résultats des tableaux II et III ci-après mettent en évidence l'intérêt du CRL 40746 en tant que vasodilatateur périphérique. En effet, ce produit est, d'une part, aussi actif que le produit de référence CP7 (LL 1656) et, d'autre part, plus efficace que ses analogues du type pipéridino tels que, en particulier, son isomère CP1 (CRL 40747) et son homologue CP2 (LL 1647) qui présente un groupe phényle trihydroxylé.

Plus précisément, le CRL 40746 selon l'invention, administré par voie I. V. et par voie I. D., augmente le débit artériel fémoral (variation toujours supérieure à + 30 %), alors que son isomère CP1 est (i) très faiblement vasodilatateur par voie I. V. (variation toujours inférieure à + 30 %, mais de l'ordre de + 20 à + 22 %) et (ii) n'a guère d'effet vasodilatateur par voie I. D. (il a plutôt un effet hypotenseur car il diminue de plus de 10 % la pression artérielle), et que son homologue trihydroxylé CP2 qui est vasodilatateur par voie I. V. (à partir de la dose de 5 mg/kg comme indiqué dans le brevet britannique n° 1.325.192 précité) est pratiquement dépourvu d'effet vasodilatateur par voie I. D. (variation du débit artériel fémoral par voie I. D. de + 17 % avec effet hypotenseur).

En clinique, le CRL 40746 a donné de bons résultats chez l'homme dans le traitement des escarres.

Tableau I

Produits testés

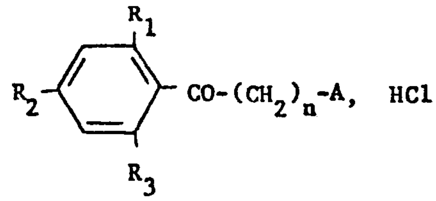

| Produit | N° de code | $R_1$ | $R_2$ | $R_3$ | n | A |
|---|---|---|---|---|---|---|
| exemple 1 | CRL 40746 | $OCH_3$ | OH | $OCH_3$ | 3 | pipéridino |
| CP1 (a) | CRL 40747 | $OCH_3$ | $OCH_3$ | OH | 3 | pipéridino |
| CP2 (b) | LL 1647 | OH | OH | OH | 3 | pipéridino |
| CP3 (c) | - | $OCH_3$ | $OCH_3$ | $OCH_3$ | 2 | pipéridino |
| CP4 (c) | - | $OCH_3$ | $OCH_3$ | $OCH_3$ | 2 | 4-méthylpipéridino |
| CP5 (d) | - | $OCH_3$ | $OCH_3$ | H | 1 | pipéridino |
| CP6 (d) | - | $OCH_3$ | $OCH_3$ | $OCH_3$ | 1 | 4-méthylpipéridino |
| CP7 (b) | LL 1656 | $OCH_3$ | $OCH_3$ | $OCH_3$ | 3 | pyrrolidino |

Notes

(a) isomère de l'exemple 1
(b) décrit dans le brevet britannique No 1 325 192
(c) décrit dans les brevets français No 1 492 256 et 5636 M
(d) décrit dans le brevet britannique No 1 115 992

Tableau II

Variation des paramètres après administration intraveineuse chez le chien anesthésié

| Produit | n° de code | Dose mg/kg | Variations en % | | |
|---|---|---|---|---|---|
| | | | Pression artérielle | Débit artériel fémoral | Fréquence cardiaque |
| Exemple 1 | CRL 40746 | 1,5 | + 2 | + 34 | + 2 |
| CP1 | CRL 40747 | 1,5 | + 2 | + 20 | + 5 |
| CP2 | LL 1647 | 1,5 | + 1 | + 10 | - 2 |
| CP3 | - | 1,5 | + 3 | + 8 | - 1 |
| CP4 | - | 1,5 | - 2 | + 3 | - 2 |
| CP5 | - | 1,5 | - 1 | - 5 | + 1 |
| CP6 | - | 1,5 | + 1 | + 7 | + 3 |
| CP7 | LL 1656 | 1,5 | 0 | + 33 | + 5 |
| Exemple 1 | CRL 40746 | 3 | + 5 | + 35 | + 3 |
| CP1 | CRL 40747 | 3 | - 12 | + 22 | - 2 |
| CP2 | LL 1647 | 3 | + 1 | + 18 | + 3 |
| CP3 | - | 3 | - 2 | + 8 | - 6 |

(suite)

| Produit | n° de code | Dose mg/kg | Variations en % | | |
|---|---|---|---|---|---|
| | | | Pression artérielle | Débit artériel fémoral | Fréquence cardiaque |
| CP4 | - | 3 | - 5 | + 2 | + 1 |
| CP5 | - | 3 | - 8 | - 8 | + 3 |
| CP6 | - | 3 | - 1 | + 5 | - 2 |
| CP7 | LL 1656 | 3 | - 2 | + 36 | + 6 |
| Exemple 1 | CRL 40746 | 6 | + 5 | + 41 | + 3 |
| CP1 | CRL 40747 | 6 | - 18 | + 22 | - 15 |
| CP2 | LL 1647 | 6 | + 2 | + 34 | + 3 |
| CP3 | - | 6 | + 5 | + 3 | - 1 |
| CP4 | - | 6 | + 1 | + 2 | - 1 |
| CP5 | - | 6 | - 2 | - 3 | + 2 |
| CP6 | - | 6 | - 8 | + 2 | + 1 |
| CP7 | LL 1656 | 6 | - 2 | + 39 | 0 |

Tableau III

Variation des paramètres après administration intraduodénale chez le chien anesthésié

| Produit | n° de code | Dose mg/kg | Variations en % | | |
|---|---|---|---|---|---|
| | | | Pression artérielle | Débit artériel fémoral | Fréquence cardiaque |
| Exemple 1 | CRL 40746 | 20 | - 2 | + 83 | - 5 |
| CP1 | CRL 40747 | 20 | - 15 | + 10 | - 2 |
| CP2 | LL 1647 | 20 | - 12 | + 17 | - 1 |
| CP3 | - | 20 | - 8 | - 5 | - 2 |
| CP4 | - | 20 | - 1 | - 2 | + 3 |
| CP5 | - | 20 | 0 | + 2 | + 1 |
| CP6 | - | 20 | + 6 | + 1 | + 4 |
| CP7 | LL 1656 | 20 | - 4 | + 84 | + 5 |

**Revendications** (pour les Etats contractants : BE, CH, DE, FR, GB, IT, LI, LU, NL, SE)

1. Nouveau dérivé appartenant à la famille des (alkoxy et hydroxy-phényl)-(3-aminopropyl)-cétones, caractérisé en ce qu'il est choisi parmi l'ensemble constitué par

(i) la (2,6-diméthoxy-4-hydroxyphényl)-(3-pipéridino-propyl)-cétone de formule :

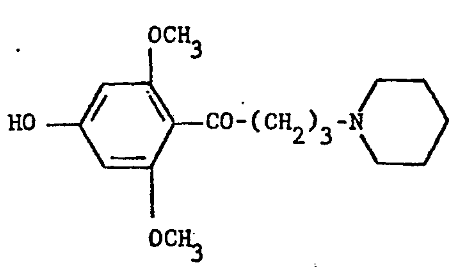

(I)

et

(ii) ses sels d'addition.

2. Chlorhydrate de (2,6-diméthoxy-4-hydroxyphényl)-(3-pipéridinopropyl)-cétone.

3. Procédé de préparation de la (2,6-diméthoxy-4-hydroxyphényl)-(3-pipéridinopropyl)-cétone, caractérisé en ce que l'on fait réagir stœchiométriquement le 1,5-diméthoxy-3-hydroxybenzène de formule :

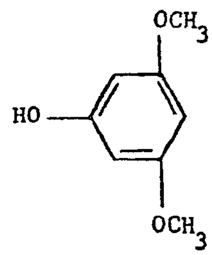

(II)

avec le pipéridinobutyronitrile de formule :

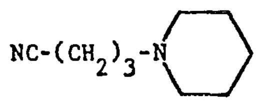

(III)

en présence de $AlCl_3$ et en faisant passer au sein du milieu réactionnel pendant au moins 2 h à une température de 10-20 °C un courant gazeux de HCl, puis hydrolyse le dérivé cétimine ainsi formé et qui a pour formule :

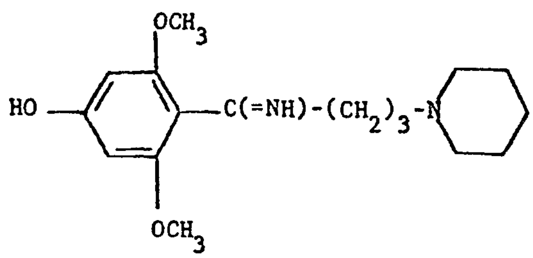

(IV)

4. Composition thérapeutique, caractérisée en ce qu'elle renferme, en association avec un excipient physiologiquement acceptable, un ingrédient actif choisi parmi la (2,6-diméthoxy-4-hydroxyphényl)-(3-pipéridinopropyl)-cétone et ses sels d'addition.

**Revendication** (pour l'Etat contractant AT)

Procédé de préparation de la (2,6-diméthoxy-4-hydroxyphényl)-(3-pipéridinopropyl)-cétone de formule développée

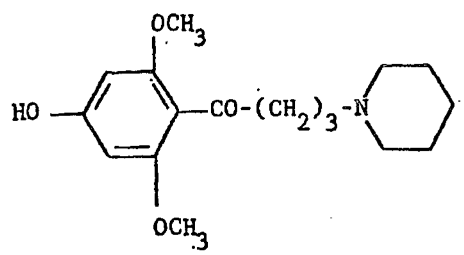

(I)

et de ses sels d'addition, caractérisé en ce que l'on fait réagir stœchiométriquement le 1,5-diméthoxy-3-hydroxybenzène de formule

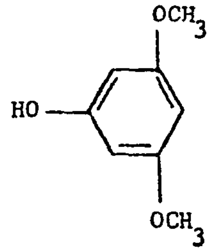

(II)

avec le pipéridinobutyronitrile de formule :

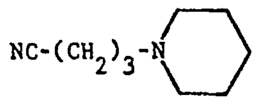

(III)

en présence de $AlCl_3$ et en faisant passer au sein du milieu réactionnel pendant au moins 2 h à une température de 10-20 °C un courant gazeux de HCl, puis hydrolyse le dérivé cétimine ainsi formé et qui a pour formule

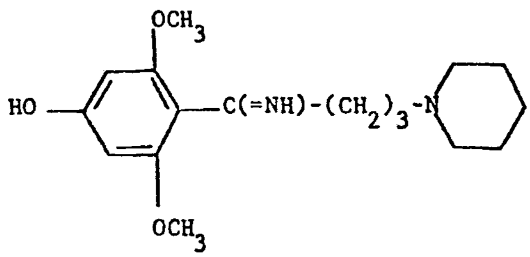

(IV)

**Claims** (for the Contracting States : BE, CH, DE, FR, GB, IT, LI, LU, NL, SE)

1. New derivative belonging to the family of (alkoxy and hydroxy-phenyl)-(3-aminopropyl) ketones, characterized in that it is selected from the group constituted by
(i) the (2,6-dimethoxy-4-hydroxyphenyl)-(3-piperidinopropyl)-ketone of formula :

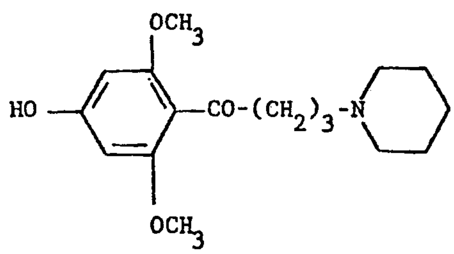

(I)

and
(ii) its addition salts.
2. (2,6-dimethoxy-4-hydroxyphenyl)-(3-piperidinopropyl)-ketone hydrochloride.
3. A method for preparing (2,6-dimethoxy-4-hydroxyphenyl)-(3-piperidinopropyl)-ketone characterized in that 1,5-dimethoxy-3-hydroxybenzene of the formula

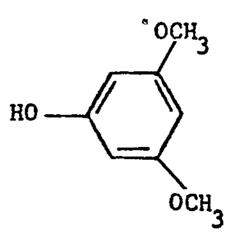

(II)

is stoichiometrically reacted with piperidinobutyronitrile of the formula

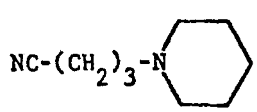

(III)

in the presence of $AlCl_3$ and by passing through the reaction medium a HCl gas stream for at least 2 hours, at 10-20 °C, then hydrolyzing the ketimine derivative thus formed and which corresponds to the formula

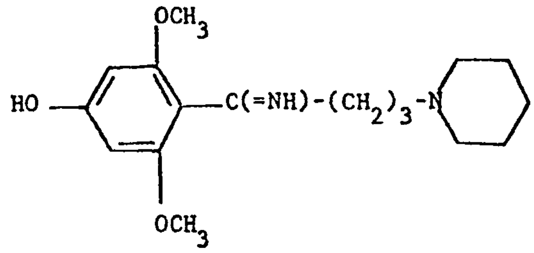

(IV)

4. A therapeutical composition, characterized in that it comprises, in association with a physiologically acceptable excipient, an active ingredient selected from (2,6-dimethoxy-4-hydroxyphenyl)-(3-piperidinopropyl)-ketone and its addition salts.

**Claim** (for the Contracting State AT)

Process for the preparation of the (2,6-dimethoxy-4-hydroxyphenyl)-(3-piperidinopropyl)-ketone of developed formula :

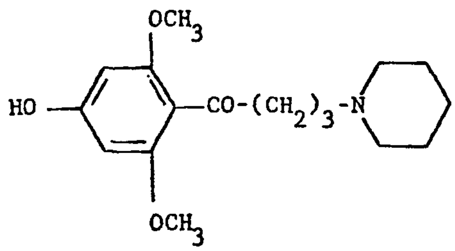

(I)

and its addition salts, characterized in that 1,5-dimethoxy-3-hydroxybenzene of the formula

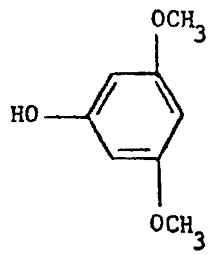

(II)

is stoichiometrically reacted with piperidinobutyronitrile of the formula

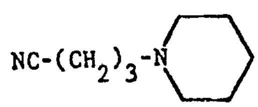

(III)

in the presence of $AlCl_3$ and by passing through the reaction medium a HCl gas stream for at least 2 hours, at 10-20 °C, then hydrolyzing the ketimine derivative thus formed and which corresponds to the formula

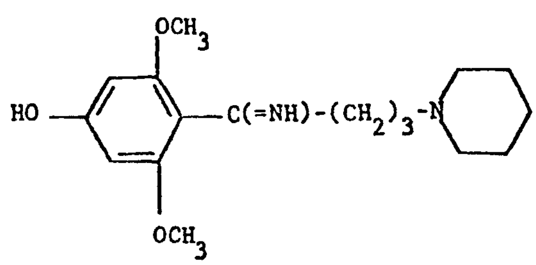

(IV)

**Ansprüche** (für die Vertragsstaaten : BE, CH, DE, FR, GB, IT, LI, LU, NL, SE)

1. Neues, zur Familie der (Alkoxy- und Hydroxy-phenyl)-(3-aminopropyl)-ketone gehörendes Derivat,

dadurch gekennzeichnet, daß es ausgewählt ist aus der Gruppe bestehend aus
(i) (2,6-Dimethoxy-4-hydroxyphenyl)-(3-piperidinopropyl)-keton der Formel

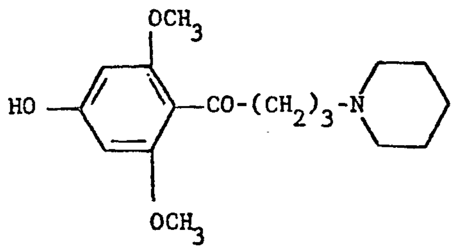

(I)

und
(ii) seinen Additionssalzen.

2. Hydrochlorid von (2,6-Dimethoxy-4-hydroxyphenyl)-(3-piperidinopropyl)-keton.

3. Verfahren zur Herstellung von (2,6-Dimethoxy-4-hydroxyphenyl)-(3-piperidinopropyl)-keton, dadurch gekennzeichnet, daß man 1,5-Dimethoxy-3-hydroxy-benzol der Formel

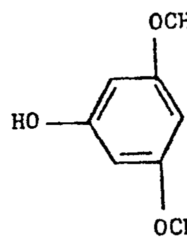

(II)

mit Piperidinobutyronitril der Formel

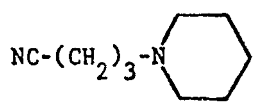

(III)

in stöchiometrischem Verhältnis in Gegenwart von $AlCl_3$ reagieren läßt, wobei ein Strom gasförmiger HCl während wenigstens 2 h bei einer Temperatur von 10 bis 20 °C durch das Reaktionsmedium geleitet wird, worauf man das so gebildete Ketimin-Derivat der Formel

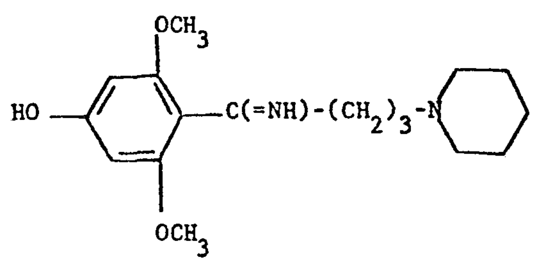

(IV)

hydrolysiert.

4. Therapeutische Zusammensetzung, dadurch gekennzeichnet, daß sie gemeinsam mit einem physiologisch akzeptablen Exzipienten ein aktives Ingredienz, ausgewählt aus (2,6-Dimethoxy-4-hydroxyphenyl)-(3-piperidinopropyl)-keton und dessen Additionssalzen umfaßt.

**Anspruch** (für den Vertragsstaat AT)

Verfahren zur Herstellung von (2,6-Dimethoxy-4-hydroxyphenyl)-(3-piperidinopropyl)-keton der Strukturformel

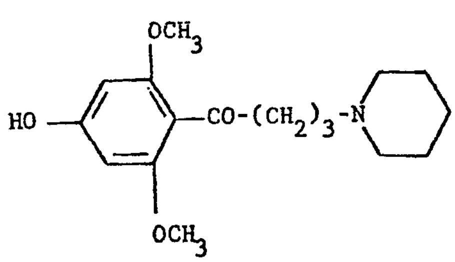

(I)

und von dessen Additionssalzen, dadurch gekennzeichnet, daß man 1,5-Dimethoxy-3-hydroxybenzol der Formel

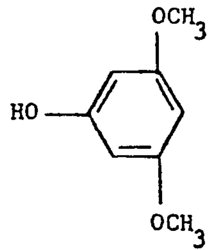

(II)

mit Piperidinobutyronitril der Formel

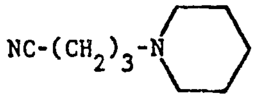

(III)

in stöchiometrischem Verhältnis in Gegenwart von $AlCl_3$ reagieren läßt, wobei ein Strom gasförmiger HCl während wenigstens 2 h bei einer Temperatur von 10 bis 20 °C durch das Reaktionsmedium geleitet wird, worauf man das so gebildete Ketimin-Derivat der Formel

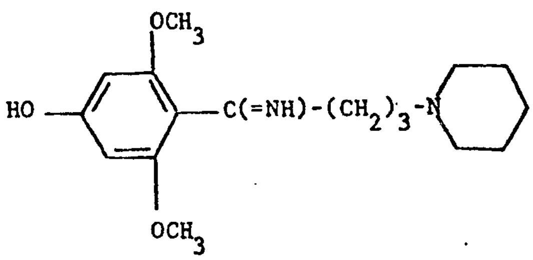

(IV)

hydrolysiert.